# EUROPEAN PATENT APPLICATION

(11) **EP 4 424 288 A1**
(43) Date of publication of application: **04.09.2024**
(21) Application number: 22886527.5
(22) Date of filing: 20.09.2022
(51) Int. Cl.: A61F 13/47, A61F 13/49, A61F 13/532, A61F 13/533

(54) **ABSORBENT ARTICLE**

(30) Priority: 28.10.2021 JP 2021176394
(71) Applicant: DAIO PAPER CORPORATION, Shikokuchuo-shi Ehime 799-0492 (JP)
(72) Inventor: TAKAHIRA, Akira, Sakura-shi, Tochigi 329-1411 (JP)
(74) Representative: Epping - Hermann - Fischer
(86) International application number: PCT/JP2022/035035
(87) International publication number: WO 2023/074193

(57) **Abstract**

An absorbent article includes a liquid-permeable top sheet; a liquid-impermeable back sheet; and an absorbent provided between the top sheet and the back sheet, the absorbent article having a longitudinal direction and a widthwise direction orthogonal to the longitudinal direction. The absorbent article includes a center compressed groove extending along the longitudinal direction in a center region in the widthwise direction, and a pair of oblique compressed grooves that are each connected to one end of the center compressed groove in the longitudinal direction and are extended such that a distance between the pair of oblique compressed grooves increases outward in the longitudinal direction. A length of the oblique compressed grooves in the longitudinal direction is 30% or more a length of the center compressed groove in the longitudinal direction.

## Description

### TECHNICAL FIELD

The present invention relates to absorbent articles.

Pad-shaped absorbent articles, such as pads for incontinence, sanitary napkins, pad-type diapers, and the like, are used in a state in which they are three-dimensionally deformed to conform to the body shape of a wearer upon wearing thereof. If the absorbent article is not properly deformed, unwanted wrinkles, twists, and the like may be formed, and the absorbent article may not be able to fully exhibit functions thereof. In view of this, various studies have been made on a configuration in which the absorbent article is properly deformed.

For example, PTL 1 describes an absorbent article including: a pair of forward bending guide portions 81 on both outer sides with respect to a widthwise center in an excretory outlet region ER, the pair of forward bending guide portions 81 extending rearward from a position away from a front edge 30F of an absorbent; and a rearward bending guide portion 82 disposed between the pair of forward bending guide portions 81 and extending in a forward-rearward direction L rearward of the excretory outlet region ER. According to the description of PTL 1, this configuration makes the absorbent article readily fit a wearer's body from the excretory outlet through the perineum until the buttock.

### CITATION LIST

### PATENT LITERATURE

PTL 1: Japanese Patent Application Publication No. 2021-97854

### SUMMARY OF INVENTION

### TECHNICAL PROBLEM

In the absorbent article described in PTL 1, desired deformation may not be achieved, for example, because the forward bending guide portions 81 are separated from the rearward bending guide portion 82. Especially, a deformed state of an overlapping region OR between the forward bending guide portions 81 and the rearward bending guide portion 82 may differ each time in accordance with, for example, how the absorbent article is attached or worn. Thus, there is possibility that wrinkles or twists will occur at an undesired site and body fluid may leak from that site.

In view of the above, in one aspect of the present invention, it is an object to provide an absorbent article that is readily deformable into a desired shape upon wearing of the absorbent article and is reduced in formation of wrinkles or twists at an undesired site.

### SOLUTION TO PROBLEM

One aspect of the present invention relates to an absorbent article including: a liquid-permeable top sheet; a liquid-impermeable back sheet; and an absorbent provided between the top sheet and the back sheet, the absorbent article having a longitudinal direction and a widthwise direction orthogonal to the longitudinal direction. The absorbent article includes a center compressed groove extending along the longitudinal direction in a center region in the widthwise direction, and a pair of oblique compressed grooves that are each connected to one end of the center compressed groove in the longitudinal direction and are extended such that a distance between the pair of oblique compressed grooves increases outward in the longitudinal direction. A length of the oblique compressed grooves in the longitudinal direction is 30% or more a length of the center compressed groove in the longitudinal direction.

### ADVANTAGEOUS EFFECTS OF INVENTION

According to one aspect of the present invention, it is possible to provide an absorbent article that is readily deformable into a desired shape upon wearing of the absorbent article and is reduced in formation of wrinkles or twists at an undesired site.

### BRIEF DESCRIPTION OF DRAWINGS

[FIG. 1] FIG. 1 is a plan view of an absorbent article according to a first embodiment as viewed from a skin side.
[FIG. 2] FIG. 2 is a cross-sectional view of FIG. 1 as taken along line I-I in FIG. 1.
[FIG. 3] FIG. 3 is a cross-sectional view of FIG. 1 as taken along line II-II in FIG. 1.
[FIG. 4] FIG. 4 is a partially enlarged view of a compressed groove in the absorbent article as illustrated in FIG. 1.
[FIG. 5] FIG. 5 is a perspective view illustrating a deformed state of the absorbent article according to the first embodiment.
[FIG. 6] FIG. 6 is a plan view of an absorbent article according to a second embodiment as viewed from the skin side.
[FIG. 7] FIG. 7 is an enlarged view of compressed grooves of the absorbent article as illustrated in FIG. 6.

### DESCRIPTION OF EMBODIMENTS

In the following, embodiments of the present invention will be described with reference to the drawings. In the drawings, unless otherwise specified, the same or corresponding components are denoted by the same symbols, and description thereof may be omitted.

### <First Embodiment>

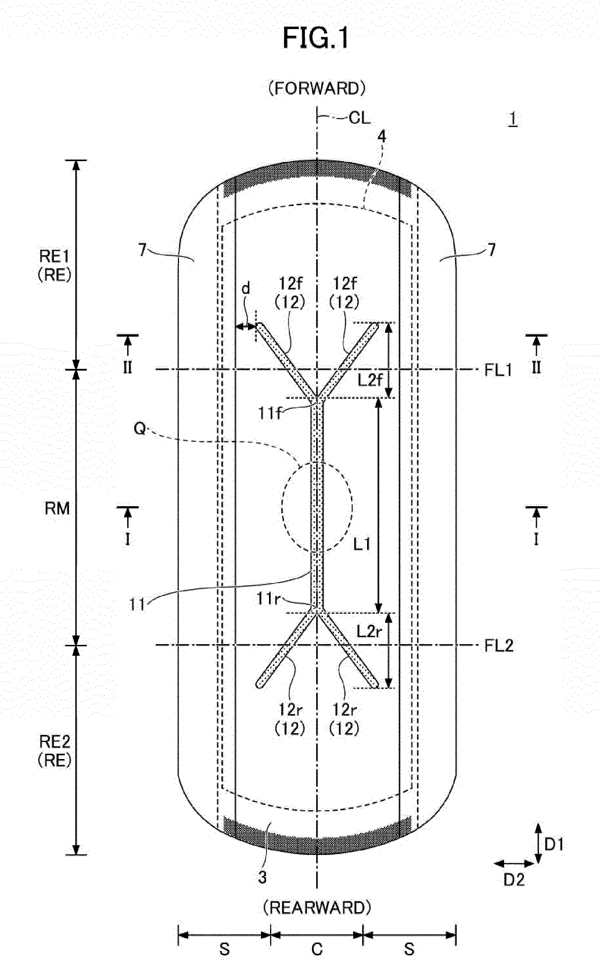

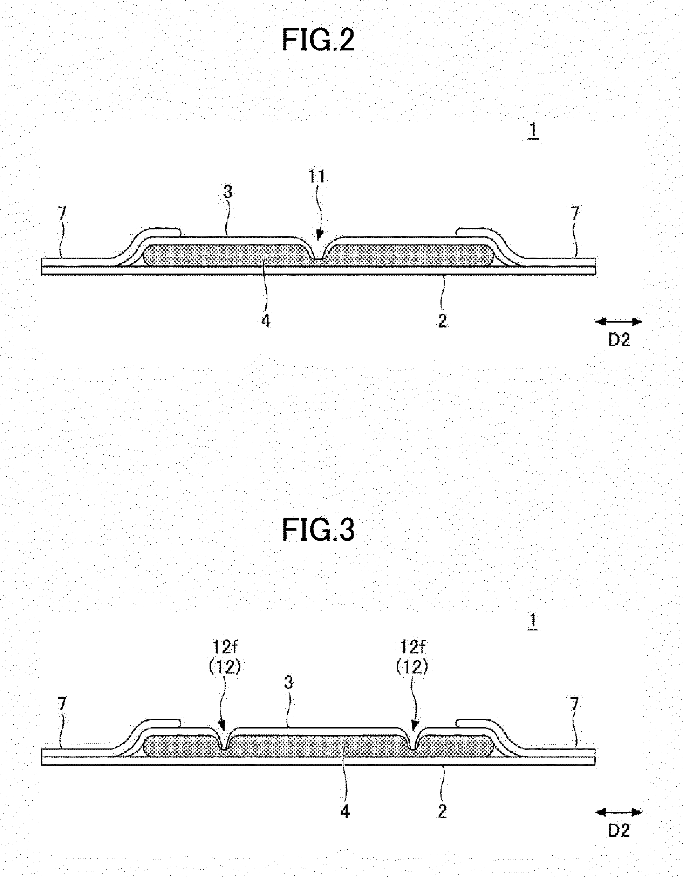

FIG. 1 is a plan view of an absorbent article 1 according to the first embodiment of the present invention. This example is a pad for incontinence. FIG. 2 is a cross-sectional view of the absorbent article 1 as taken along line I-I, and FIG. 3 is a cross-sectional view of the absorbent article 1 as taken along line II-II. As illustrated in FIG. 1 to FIG. 3, the absorbent article 1 is a stack of a liquid-permeable top sheet 3, a liquid-impermeable back sheet 2, and an absorbent 4 disposed between both of the sheets. The absorbent article 1 has an approximately flat shape as a whole in a state before wearing thereof (FIG. 2 and FIG. 3). When the absorbent article 1 is worn, a top sheet 3 side becomes a skin-touched side (skin side or front side) and a back sheet 2 side becomes an underwear-fixed side (underwear side or back side). Therefore, FIG. 1 is a view of the absorbent article 1 from the skin side.

The absorbent article 1 may have an elongated shape in a plan view, i.e., a shape having a predetermined length in a longitudinal direction D1 and a predetermined width in a widthwise direction D2 orthogonal to the longitudinal direction D1. Upon wearing of the absorbent article 1, the longitudinal direction D1 of the absorbent article 1 corresponds to a forward-rearward direction of a wearer's body, and the widthwise direction D2 corresponds to a leftward-rightward direction of the wearer's body. In the form as illustrated in FIG. 1, the absorbent article 1 has, in a plan view, a shape that is approximately in line symmetry with respect to a centerline (longitudinal centerline) CL extending in the longitudinal direction D1. However, the absorbent article 1 needs not be a line-symmetric shape. The configurations other than the shape of the absorbent article 1 (including the density and material of the absorbent 4, the size of the compressed groove, and the like) may be approximately symmetrical or may be asymmetrical with the centerline CL as an axis of symmetry.

The back sheet 2 may be a sheet having at least water impermeability and may be, for example, a sheet formed of an olefin resin, such as polyethylene, polypropylene, or the like. Also, the back sheet 2 may be, for example, a laminated nonwoven fabric in which a nonwoven fabric is laminated on a polyethylene sheet or the like, or a laminated sheet of a nonwoven fabric in which a waterproof film is interposed to substantially ensure liquid impermeability. In addition, it is more desirable to use one having moisture permeability from the viewpoint of preventing moisture-induced discomfort. As such a water-impermeable and moisture-permeable sheet material, it is possible to use a microporous sheet or the like obtained by forming a sheet through melt-kneading an inorganic filler in an olefin-based resin, such as polyethylene, polypropylene, or the like, and then stretching the resulting product in a uniaxial or biaxial direction.

The top sheet 3 may be a sheet that is permeable to body fluid, such as urine, menstrual blood, vaginal discharge, and the like. As the top sheet 3, porous or non-porous non-woven fabric, a porous plastic sheet, or the like is preferably used. As the material fibers that form the non-woven fabric, for example, olefins, such as polyethylene, polypropylene, and the like, synthetic fibers, such as polyester, polyamide, and the like, regenerated fibers, such as rayon, cupra, and the like, blended fibers thereof, and natural fibers, such as cotton and the like, may be used alone or in combination. Further, examples of a method for processing a nonwoven fabric include a spunlace method, a spunbond method, a thermal bond method, a melt blown method, a needle punch method, and the like. Of these processing methods, a spunlace method is preferable in terms of flexibility, a spunbond method is preferable because a nonwoven fabric with high drape properties can be produced, and a thermal bond method is preferable because a soft nonwoven fabric with high bulkiness can be produced. Moreover, composite fibers including core-in-sheath fibers having a high-melting-point fiber as a core and a low-melting-point fiber as a sheath, side-by-side fibers, split fibers, and the like may be used.

No particular limitation is imposed on a material of the absorbent 4 as long as the material can absorb and retain body fluid. However, the absorbent 4 preferably includes cotton-like pulp and a water-absorbing polymer. Examples of the water-absorbing polymer that may be used include superabsorbent polymers (SAPs), superabsorbent fibers (SAFs), and combinations thereof. Examples of the pulp include cellulose fibers, such as chemical pulp, dissolving pulp, and the like, obtained from wood, and artificial cellulose fibers, such as rayon, acetate, and the like. Hardwood materials, softwood materials, and the like are used as raw materials for the chemical pulp. However, softwood materials are preferably used in view of their long fiber length and the like.

A synthetic fiber may be mixed with the absorbent 4. Examples of the synthetic fiber that may be used include: polyolefins, such as polyethylene, polypropylene, and the like; polyesters, such as polyethylene terephthalate, polybutylene terephthalate, and the like; polyamides, such as nylon and the like; and copolymers thereof. Two or more of these materials may also be used. Further, composite fibers including core-in-sheath fibers having a high-melting-point fiber as a core and a low-melting-point fiber as a sheath, side-by-side fibers, split fibers, and the like may be used. Hydrophobic fibers that are surface-treated with a hydrophilizing agent to exhibit affinity to body fluid may also be used. The absorbent 4 is preferably produced by a fiber stacking method or an air laid method.

The absorbent 4 may be obtained by enclosing a main body portion thereof with an encapsulating sheet formed of a crepe paper, a nonwoven fabric, or the like. Because the absorbent 4 includes the encapsulating sheet, it is possible to prevent the absorbent 4 from being twisted or split, and to retain the shape. As the encapsulating sheet, a crepe paper or nonwoven fabric that is uncolored (i.e., white) or colored (e.g., colored in a color like the color of the body fluid or a complementary color of the color of the body fluid) may be used.

As illustrated in FIG. 1, the absorbent 4 may have an elongated shape having an approximately constant width in a plan view. However, the width of the absorbent 4 may vary in the longitudinal direction D1. The absorbent 4 as a whole may have a uniform thickness over the entire surface, but the thickness of the absorbent 4 may not be uniform, and may be locally thin or thick.

At both end portions of the absorbent 4 in the longitudinal direction D1, end portions of the back sheet 2 and the top sheet 3 may be bonded to each other with an adhesive, heat sealing, or the like. On both side portions of the absorbent article 1, i.e., on both sides in the width direction D2, a pair of side sheets 7 and 7 are disposed on the front side (top sheet 3 side) in the longitudinal direction D1.

The side sheet 7 may be formed by using a nonwoven fabric material subjected to an appropriate water-repellent treatment or a hydrophilic treatment in accordance with the intended purpose, such as preventing permeation of body fluid, enhancing the texture on the skin, or the like. The material of the side sheet 7 may be a natural fiber, a synthetic fiber, a regenerated fiber, or the like. When the side sheet 7 is subjected to a water-repellent treatment, a water-repellent agent, such as a silicone-based agent or a paraffin-based agent, can be used for the treatment. Note that the absorbent article may have a configuration in which the top sheet 3 extends to the end portions of the absorbent article 1 in the width direction D2 and is bonded to the back sheet 2 without using the side sheet 7.

The absorbent article 1 includes: a middle region RM mainly corresponding to the groin of a wearer upon wearing of the absorbent article 1; and end regions RE next to the middle region RM in the longitudinal direction D1. The end regions RE include: a forward end region RE1 that is next to a forward portion of the middle region RM and extends to a forward end of the absorbent article 1; and a rearward end region RE2 that is next to a rearward portion of the middle region RM and extends to a rearward end of the absorbent article 1. The middle region RM includes a body fluid outlet-facing region Q. The body fluid outlet-facing region Q is a region that faces body fluid outlets, such as a urinary opening, a vaginal opening, and the like of the wearer upon wearing of the absorbent article 1. The center of the body fluid outlet-facing region Q is on the longitudinal centerline CL. In the example as illustrated in FIG. 1, the body fluid outlet-facing region Q is illustrated in an oval shape as a region corresponding to the urethral opening. However, the size and shape of the body fluid outlet-facing region Q as illustrated are merely examples for describing the absorbent article according to the present embodiment.

The forward end region RE1 and the rearward end region RE2 (which may be collectively referred to simply as an end region RE) can be folded toward the skin side in the longitudinal direction D1 when the absorbent article is individually packaged. In the example of FIG. 1, folding of the forward end region RE1 can be performed at a fold line FL1 along the boundary between the forward end region RE1 and the middle region RM, and folding of the rearward end region RE2 can be performed at a fold line FL2 along the boundary between the rearward end region RE2 and the middle region RM. Thus, the absorbent article 1 can be folded in three or more. Either of the end regions, i.e., the forward end region RE1 and the rearward end region RE2, may be folded first.

As viewed in the widthwise direction D2, the absorbent article 1 may include a center region C including the longitudinal centerline CL, and side regions S and S that are next to outer ends of the center region C in the widthwise direction D2 and extend to outer ends of the absorbent article 1 in the widthwise direction D2. The body fluid outlet-facing region Q is included in the center region C. The center region C may be a portion having a width of from 30% through 40% of the width of the main body of the absorbent article 1 (excluding the wing if provided) and preferably a portion of 1/3 the width of the main body of the absorbent article 1.

The length (total length) of the entirety of the absorbent article 1 in the longitudinal direction D1 is preferably from 170 mm through 360 mm and more preferably may be from 230 mm through 270 mm. The thickness of the entirety of the absorbent article 1 is preferably from 1 mm through 30 mm and more preferably may be from 5 mm through 20 mm.

As illustrated in FIG. 1 to FIG. 3, the absorbent article 1 is provided with compressed grooves (11, 12f, 12f, 12r, 12r) that are recessed from the skin side to a non-skin side (i.e., recessed from the top sheet 3 side to the back sheet 2 side). The compressed groove can be formed by applying, from the top sheet 3 side, a pressure in a predetermined shape to a predetermined position on a laminate of at least the absorbent 4 and the top sheet 3. In this case, for example, the laminate including the absorbent 4 and the top sheet 3 may be allowed to pass between: a roll having a convex portion conforming to the shape of a predetermined compressed groove; and a roll having no irregularities on the surface facing the roll. Because the compressed groove is a groove recessed from the top sheet 3 side to the back sheet 2 side, the center region C of the absorbent article 1 is readily deformed so as to project toward the back sheet 2 side (non-skin side) in response to receiving a force from both sides in the widthwise direction D2.

The degree of compression (depth of the groove) in the compressed groove may be uniform as a whole or may vary from place to place. Although not illustrated in FIG. 1, for example, the compressed groove may include a weakly compressed portion and a strongly compressed portion that recessed deeper than the weakly compressed portion, and the strongly compressed portion may be intermittently formed in the weakly compressed portion (which will be described in detail in a second embodiment).

As illustrated in FIG. 1, the compressed groove includes: a center compressed groove 11 extending along the longitudinal direction D1 in the center region C in the widthwise direction D2; and a pair of oblique compressed grooves 12 and 12 each connected to one end of the center compressed groove 11. The pair of oblique compressed grooves 12 and 12 in the present embodiment may include either or both of: a pair of forward oblique compressed grooves 12f and 12f each connected to a forward end 11f of the center compressed groove 11; and a pair of rearward oblique compressed grooves 12r and 12r each connected to a rearward end 11r of the center compressed groove 11, as illustrated in FIG. 1. Upon wearing of the absorbent article 1, the absorbent article 1 may be folded along the center compressed groove 11 and the pair of oblique compressed grooves 12 and 12, and may be deformed three-dimensionally to conform to the body as a whole.

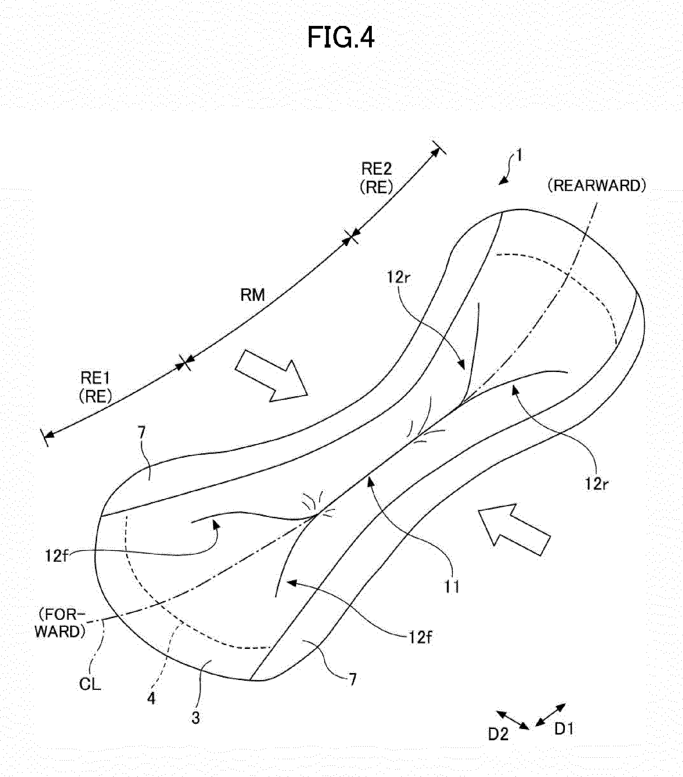

FIG. 4 illustrates an example of a state in which the absorbent article 1 is deformed upon wearing thereof. Upon wearing of the absorbent article 1, both legs are roughly positioned at both ends of the middle region RM in the widthwise direction D2. Thus, as illustrated in FIG. 4, the middle region RM is narrowed in the widthwise direction D2 by receiving a force from the legs. At this time, the center compressed groove 11 sinks toward the back sheet 2 side (projects toward the back sheet 2 side), and portions on both sides of the center compressed groove 11 come close to the longitudinal centerline CL. At this time, a recess that projects toward the non-skin side is formed in the center region in the widthwise direction D2, and body fluid (e.g., urine and the like) discharged to the recess can be retained in this space at least temporarily. Therefore, even if a large amount of body fluid is rapidly discharged, the body fluid can be absorbed by the absorbent 4 without leaking laterally from the absorbent article 1.

Upon wearing of the absorbent article 1, the absorbent article 1 is not only narrowed in the width in the middle region RM in the longitudinal direction D1, but also curved such that the forward end region RE1 and the rearward end region RE2, not corresponding to the groin, are lifted to the top sheet 3 side or such that the center of the absorbent article 1 in the longitudinal direction D1 projects toward the back sheet side. Then, a forward portion and a rearward portion of the absorbent article 1 can be made just wide enough so that the width does not become narrowed, and can be brought into contact with the wearer's body as a plane. More specifically, the forward end region RE1 can face the vicinity of the pubic bone at the front of the body, and the rearward end region RE2 can face the buttock.

As illustrated in FIG. 1, the center compressed groove 11 is formed to overlap the body fluid outlet-facing region Q in the longitudinal direction D1. Further, the center compressed groove 11 is more preferably formed to have an enough length to include the body fluid outlet-facing region Q in the longitudinal direction D1. As a result, the discharged body fluid can be quickly transferred in the longitudinal direction D1 along the center compressed groove 11, and leakage to outer sides in the widthwise direction D2 can be prevented. In addition, the center compressed groove 11 may be formed to overlap the middle region RM, and preferably to be included in the middle region RM. Although there may be a plurality of center compressed grooves 11, one of the center compressed grooves 11 is preferably formed to overlap the longitudinal centerline CL.

The center compressed groove 11 may be a compressed groove that is continuous from one end thereof to the other end thereof (from the forward end to the rearward end). Because the center compressed groove 11 is continuous, when a force is applied to the absorbent article 1 upon wearing thereof, desired deformation in which the absorbent article 1 projects toward the back sheet 2 side in the middle region RM can be guided continuously and more reliably over the entirety in the longitudinal direction D1 (FIG. 4). In an existing configuration in which a compressed groove includes a discontinuous portion, deformation is not successfully guided at the discontinuous portion, and wrinkles, twists, folds, and the like may occur at undesired sites, causing inconvenience, such as leakage of the body fluid from these sites. In the present embodiment, such inconvenience can be prevented.

In the illustrated example, the center compressed groove 11 is formed in a straight line parallel to the longitudinal centerline CL. However, the center compressed groove 11 may include a meandering portion, an obliquely extending portion, and the like. Preferably, the center compressed groove 11 is formed in a straight line because the absorbent article 1 is more readily folded along the center compressed groove 11 even by the action of a small quantity of force.

In the present embodiment, the pair of oblique compressed grooves 12 and 12 are provided in addition to the center compressed groove 11. The pair of oblique compressed grooves 12 and 12 are each connected to one end of the center compressed groove 11 in the longitudinal direction D1 and extend from the one end outward in the longitudinal direction D1. It is enough to form the pair of forward oblique compressed grooves 12f and 12f or the pair of rearward oblique compressed grooves 12r and 12r. However, both the pair of forward oblique compressed grooves 12f and 12f and the pair of rearward oblique compressed grooves 12r and 12r are preferably formed as illustrated in FIG. 1 because desired deformation of the absorbent article 1 can be guided over the entirety in the longitudinal direction D1. Preferably, the pair of oblique compressed grooves 12 and 12 are formed in line symmetry with the longitudinal centerline CL as the axis of symmetry.

The pair of oblique compressed grooves 12 and 12 extend such that the distance therebetween increases outward in the longitudinal direction D1. In the pair of forward oblique compressed grooves 12f and 12f, the distance therebetween increases forward, and in the pair of rearward oblique compressed grooves 12r and 12r, the distance therebetween increases rearward. More specifically, the pair of forward oblique compressed grooves 12f and 12f may be a pair of a compressed groove obliquely extending forward and rightward and a compressed groove obliquely extending forward and leftward, and the pair of rearward oblique compressed grooves 12r and 12r may be a pair of a compressed groove obliquely extending rearward and rightward and a compressed groove obliquely extending rearward and leftward.

Because the center compressed groove 11 is connected to the pair of oblique compressed grooves 12 and 12 as described above, a single continuous compressed groove can be formed by the center compressed groove 11 and the pair of oblique compressed grooves 12 and 12. Thus, when a force is applied to the absorbent article 1 upon wearing thereof, desired deformation can be guided along the compressed grooves over the entirety in the longitudinal direction D1. It is therefore possible to prevent undesired folds, wrinkles, twists, and the like which would otherwise be formed by the presence of a place where a folded position is not determined due to discontinuous compressed grooves. Hence, it is possible to prevent leakage of body fluid. Further, as illustrated in FIG. 1, when the pair of forward oblique compressed grooves 12f and 12f are each connected to the forward end 11f of the center compressed groove 11 and the pair of rearward oblique compressed grooves 12r and 12r are each connected to the rearward end 11r, a desired deformed state can be achieved continuously over the entirety of the absorbent article 1 in the longitudinal direction D1, which is preferable. In addition, such a deformed state can be stably retained during wearing of the absorbent article 1.

Because the distance between the pair of oblique compressed grooves 12 and 12 increases outward in the longitudinal direction, an approximately V shape can be formed by the two compressed grooves. Further, because the pair of oblique compressed grooves 12 and 12 are each connected to one end of the center compressed groove 11, a continuous Y-shaped compressed groove can be formed. With such a Y shape, the absorbent article 1 can be deformed to conform to the shape of the wearer's body, especially the inner shapes of the legs of the wearer. That is, the absorbent article 1 can be deformed such that the width of the middle region RM of the absorbent article 1 is narrowed and the width of the end region RE is not narrowed.

Because the pair of oblique compressed grooves 12 and 12 have an approximately V shape, it is possible to form a compressed groove-free, approximately triangular area between the pair of oblique compressed grooves 12 and 12 by forming no compressed groove between the pair of oblique compressed grooves 12 and 12. Such an approximately triangular area may contact, as a plane, the front (near the pubic bone) and/or the back (buttock) of the wearer's body. Such a compressed groove-free, approximately triangular area is flexible, and can be curved to conform to the shape of the front (near the pubic bone) and/or the back (buttock) of the body. In other words, a forward compressed groove-free, approximately triangular area can be curved such that the center thereof in the widthwise direction D2 projects forward of the body, and a rearward compressed groove-free, approximately triangular area can be curved such that the center thereof in the widthwise direction D2 projects rearward of the body. Thus, leakage forward and/or rearward of the body can be prevented. In addition, sensation in wearing can be improved.

The length of the pair of oblique compressed grooves 12 in the longitudinal direction D1 is preferably from 30% through 60% the length of the center compressed groove 11 in the longitudinal direction D1. More specifically, a length L2f of the forward oblique compressed groove 12f in the longitudinal direction D1 may be from 30% through 60% a length L1 of the center compressed groove 11 in the longitudinal direction D1, and/or a length L2r of the rearward oblique compressed groove 12r in the longitudinal direction D1 may be from 30% through 60% the length L1 of the center compressed groove 11 in the longitudinal direction D1. By setting a ratio of the length of the pair of oblique compressed grooves 12 in the longitudinal direction D1 to the length of the center compressed groove 11 in the longitudinal direction D1 to 30% or more, the pair of oblique compressed grooves 12 and 12 can have a sufficient length in the longitudinal direction D1, can form an appropriately sized Y shape with the center compressed groove 11, and can readily and naturally deform the absorbent article 1 to conform to inner portions of the legs of the wearer. In addition, an approximately triangular area that is sufficiently large can be ensured between the pair of oblique compressed grooves 12 and 12, improving the effect that this area can contact, as a plane, the body in conformity to the shape of the front and/or rear of the body. Meanwhile, by setting the above ratio to 60% or less, it is possible to avoid occurrence of folding at a position close to the forward and/or rearward end in the longitudinal direction D1, and to allow the forward portion and/or the rearward portion of the absorbent article 1 to firmly contact the body.

The pair of oblique compressed grooves 12 and 12 may be formed in a range where the absorbent 4 is disposed in a plan view, and preferably in a range where the top sheet 3 is exposed in a plan view. That is, preferably, the pair of oblique compressed grooves 12 and 12 do not overlap the side sheets 7. Further, the pair of oblique compressed grooves 12 are more preferably formed away by a distance d from the inner ends of the side sheets 7 in the widthwise direction D2 (the ends closer to the longitudinal centerline CL). Thereby, it is possible to prevent leakage of body fluid caused by deformation along the compressed grooves at the ends of the absorbent article 1 in the widthwise direction D2. In addition, it is possible to prevent the forward portion and/or the rearward portion of the absorbent article 1 from becoming harder near the ends in the widthwise direction D2 so that the forward portion and/or the rearward portion of the absorbent article 1 are not readily curved. The distance d is preferably from 5 mm through 10 mm.

Further, the total length of the entire compressed groove in the longitudinal direction D1, i.e., the sum of the length L2f of the forward oblique compressed groove 12f in the longitudinal direction D1, the length L1 of the center compressed groove 11 in the longitudinal direction D1, and the length L2r of the rearward oblique compressed groove 12r in the longitudinal direction D1 (L2f + L1 + L2r) may be from 40% through 75% of the length of the absorbent 4 in the longitudinal direction D1. Thereby, desired deformation of the absorbent article 1 can be more reliably achieved. It is therefore possible to prevent formation of wrinkles, twists, and the like at undesired sites, and also prevent inhibition of deformation conforming to the shapes of the legs due to extension of the compressed groove along the forward-rearward direction of the body.

Although FIG. 1 illustrates the positions of folding lines FL1 and FL2 for folding, the positions of FL1 and FL2 are not limited to the illustrated positions. For example, the folding line FL1 may not necessarily be formed at the boundary between the forward end region RE1 and the middle region RM, and the folding line FL2 may not necessarily be formed at the boundary between the middle region RM and the rearward end region RE2. However, preferably, the folding line FL1 and/or the folding line FL2 do not cross the center compressed groove 11, and more preferably, both of the folding lines FL1 and FL2 do not cross the center compressed groove 11. Thereby, it is possible to reduce or eliminate possibility that the folding lines FL1 and FL2 form a folded trace in a direction crossing the center compressed groove 11, and the folded trace prevents transfer of body fluid along the center compressed groove 11.

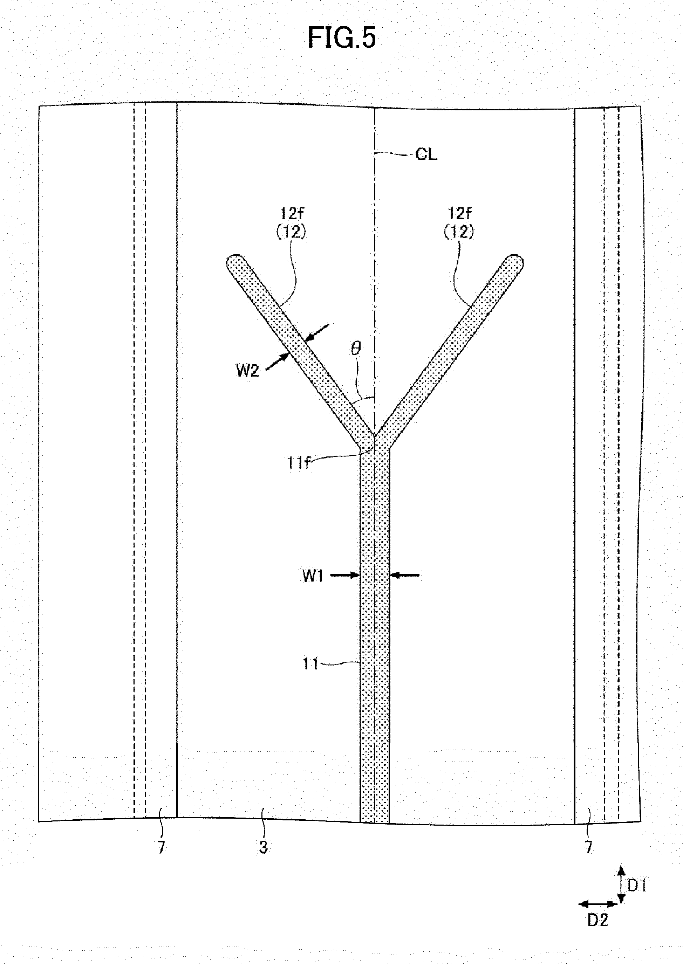

FIG. 5 is a partially enlarged view of the center compressed groove 11 and the pair of the forward oblique compressed grooves 12f and 12f. As illustrated in FIG. 5, the forward oblique compressed grooves 12f and 12f are each connected to the forward end 11f of the center compressed groove 11. In other words, the forward oblique compressed grooves 12f and 12f are branched from the forward end 11f of the center compressed groove 11. An angle θ formed between the forward oblique compressed groove 12f (oblique compressed groove 12) and the longitudinal centerline CL is preferably from 20° through 60° and more preferably from 30° through 45°. By setting the angle θ in the above range, natural deformation conforming to the inner shapes of the legs can be achieved. The angle θ may be an angle formed between the inner outline of the forward oblique compressed groove 12f (oblique compressed groove 12) in the widthwise direction D2 and the longitudinal centerline CL. When the pair of oblique compressed grooves 12 are curved, the angle θ may be an angle formed between the inner outline of the oblique compressed groove 12 in the widthwise direction D2 and the longitudinal centerline CL at the connection between the oblique compressed groove 12 and the center compressed groove 11.

As illustrated in FIG. 5, a width W2 of the oblique compressed groove is preferably smaller than a width W1 of the center compressed groove 11. Thereby, the center compressed groove 11 can be made thicker, and folding along the center compressed groove 11 can be more readily achieved. Further, because the oblique compressed groove 12 can be formed thinner, it is possible to ensure flexibility of the end region RE of the absorbent article in which the oblique compressed groove 12 is formed (i.e., a portion that is intended to contact the front portion and/or the back portion of the body in a relatively large area upon wearing of the absorbent article). The width W2 of the oblique compressed groove is preferably from 20% through 50% the width W1 of the center compressed groove 11.

The width W1 of the center compressed groove 11 may be from 3 mm through 15 mm. By setting the width W1 in the above range, it is possible to readily deform the middle region RM, among others, and to prevent the center region C in the widthwise direction D2 from becoming harder and causing discomfort felt, for example, when the legs are closed. Further, the width W2 of the oblique compressed groove 12 may be from 1 mm through 4 mm. By setting the width W2 in the above range, desired deformation conforming to the body shape can be achieved more reliably. In addition, it is possible to prevent a portion in which the oblique compressed grooves 12 and 12 are formed, from becoming harder and causing discomfort.

In the first embodiment (FIG. 1 to FIG. 4), the forward oblique compressed grooves 12f and 12f and the rearward oblique compressed grooves 12r and 12r have a relationship in which arrangements thereof are in reverse in the longitudinal direction D1, i.e., they are in line symmetry about a symmetry axis that is a virtual line in the widthwise direction D2 passing through the center of the center compressed groove 11 in the longitudinal direction D1. However, they may not necessarily be in line symmetry. For example, the angle (θ) formed between the rearward oblique compressed groove 12r and the longitudinal centerline CL may be larger or smaller than the angle formed between the forward oblique compressed groove 12f and the longitudinal centerline CL.

### <Second Embodiment>

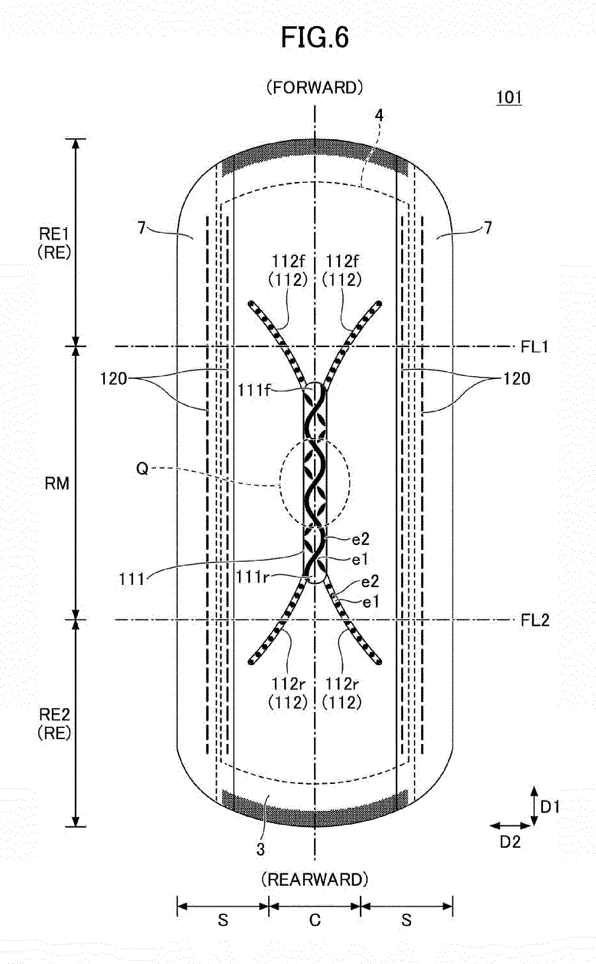

FIG. 6 is a plan view of an absorbent article 101 according to the second embodiment as viewed from the top sheet 3 side. Although the basic structure and functions of the absorbent article 101 are the same as those of the absorbent article 1 (FIG. 1 to FIG. 5), the shape of a compressed groove in the absorbent article 101 is different from the shape of the compressed groove in the absorbent article 1.

In the present embodiment, the compressed groove may include a center compressed groove 111 and a pair of oblique compressed grooves 112 and 112 each connected to one end of the center compressed groove 111. Similar to the absorbent article 1, the pair of oblique compressed grooves 112 and 112 may include a pair of forward oblique compressed grooves 112f and 112f and/or a pair of rearward oblique compressed grooves 112r and 112r. However, the pair of oblique compressed grooves 112 and 112 (the pair of forward oblique compressed grooves 112f and 112f and/or the pair of rearward oblique compressed grooves 112r and 112r) in the present embodiment do not extend in the form of a straight line, but are curved to be convex with respect to the longitudinal centerline CL. The curvature of the pair of oblique compressed grooves 112 and 112 allows the absorbent article 101 to deform more naturally to conform to curved inner shapes of the legs.

FIG. 6 illustrates a weakly compressed portion e1 and a strongly compressed portion e2 that is deeper than in the weakly compressed portion e1 by compression at a higher pressure than in the weakly compressed portion e1. In the drawing, the weakly compressed portion e1 is shown in white, and the strongly compressed portion e2 is shown in black. More specifically, the center compressed groove 111 includes the weakly compressed portion e1 and the strongly compressed portion e2 and/or the pair of oblique compressed grooves 112 and 112 include the weakly compressed portion e1 and the strongly compressed portion e2.

As illustrated in FIG. 6, the weakly compressed portion e1 is continuously formed over the pair of forward oblique compressed grooves 112f and 112f, the center compressed groove 111, and the pair of rearward oblique compressed grooves 112r and 112r. The strongly compressed portion e2 may be discontinuously formed in the region where the weakly compressed portion e1 is formed. By forming a compressed groove as a combination of the weakly compressed portion e1 and the strongly compressed portion e2, deformation along the compressed groove can be readily guided, while the absorbent 4 can be prevented from becoming excessively hard. That is, the strongly compressed portion e2 with a higher extent of compression serves as a base point for folding and facilitates folding at the compressed groove. In addition, by the presence of a shallow compressed portion between the strongly compressed portions e2, it is possible to ensure flexibility in the compressed groove.

In the example as illustrated in FIG. 6, the center compressed groove 111 includes a combination of one wavy strongly compressed portion e2 and a plurality of strongly compressed portions e2 formed apart from the wavy strongly compressed portion e2. Meanwhile, the forward oblique compressed groove 112f and the rearward oblique compressed groove 112r include the weakly compressed portion e1, and a plurality of circular strongly compressed portions e2 that are disposed along the weakly compressed portion e1 and spaced from each other.

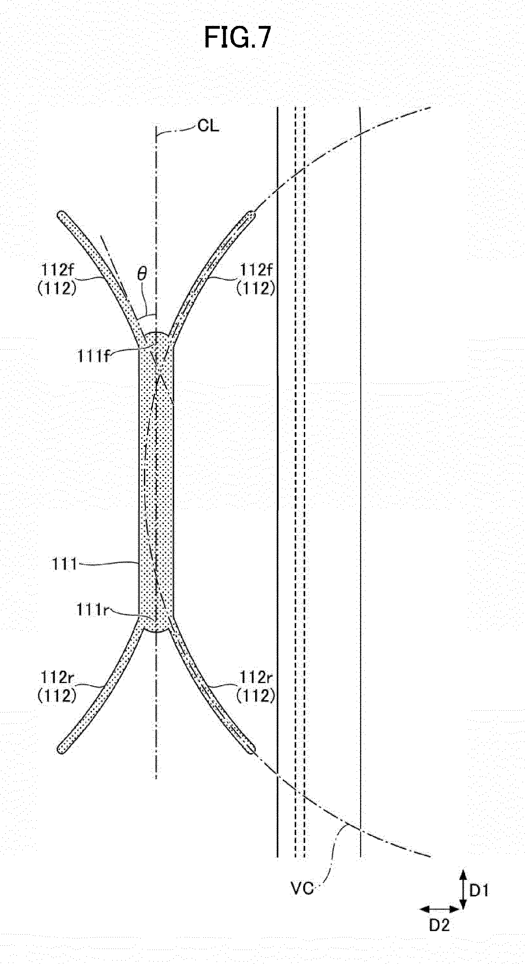

FIG. 7 is a partially enlarged view of the compressed grooves of FIG. 6. In FIG. 7, illustration of the strongly compressed portions is omitted, and a portion of the whole compressed groove (only the portion enclosed by the outline of the weakly compressed portion) is shown in gray. As described above, the pair of forward oblique compressed grooves 112f and 112f and the pair of rearward oblique compressed grooves 112r and 112r are curved to be convex with respect to the longitudinal centerline CL. Here, the curved shape of the oblique compressed groove 112 (the forward oblique compressed groove 112f and/or the rearward oblique compressed groove 112r) is preferably along the outline of an imaginary circle VC having a predetermined radius. That is, the groove centerline of at least a part of or preferably the entirety of the oblique compressed groove 112 may be along the outline of the imaginary circle VC or may form a part of the outline of the imaginary circle. The curved shape of the oblique compressed groove 112 allows the forward and/or backward deformation of the absorbent article to conform to the inner roundness of the legs of the wearer, resulting in excellent sensation in wearing.

Also, as illustrated in FIG. 7, when both of the forward oblique compressed groove 112f and the rearward oblique compressed groove 112r have a shape along the outline of the imaginary circle VC having the predetermined radius, forward and rearward deformation of the absorbent article can conform to the thickness of the leg of the wearer, which is preferable. In this case, the radius of the outline of the imaginary circle VC along which the forward oblique compressed groove 112f is may be the same as or different from the radius of the outline of the imaginary circle along which the rearward oblique compressed groove 112r is. The position of the center of the imaginary circle along which the forward oblique compressed groove 112f is may be the same as or different from the position of the center of the imaginary circle along which the rearward oblique compressed groove 112r is.

Further, as illustrated in FIG. 7, more preferably, on one side of the longitudinal centerline CL, the forward oblique compressed groove 112f and the rearward oblique compressed groove 112r are along the outline of a single identical imaginary circle VC having the predetermined radius. In other words, when a groove centerline is drawn in the forward oblique compressed groove 112f and a groove centerline is drawn in the rearward oblique compressed groove 112r, and both of the groove centerlines are extended, they can be continuously along the outline of the imaginary circle VC having the predetermined radius. This facilitates deformation that can conform to the rounded inner shapes of the legs of the wearer over the entirety in the longitudinal direction of the absorbent article.

The above predetermined radius of the imaginary circle VC may be preferably from 70 mm through 100 mm and more preferably from 80 mm through 90 mm. This allows deformation of the absorbent article to conform to the thickness of a typical adult female's leg and allows many users to feel improved sensation in wearing.

The imaginary circle VC is preferably included in the center compressed groove 111 as illustrated in FIG. 7. That is, the center compressed groove 111 is preferably formed to have an enough width to include the imaginary circle VC. Thereby, deformation in the middle region RM of the absorbent article 1 is readily achieved.

In the example as illustrated in FIG. 6, stretchable members (e.g., rubber yarns) 120 and 120 are disposed along the longitudinal direction D1 on both sides of the absorbent article 101. In the present example, two stretchable members 120 and 120 are disposed on one side of the absorbent article 101. However, the number of the stretchable members is not limited to the number illustrated. In the present embodiment, the side sheet 7 is folded back, or two or more side sheets 7 are provided on one side and the stretchable members 120 and 120 are placed between the side sheets 7. The stretchable members 120 and 120 can be fixed to the side sheets 7 in a stretched state. Therefore, a gather can be formed when the stretchable members 120 and 120 return to an initial length thereof (a state in which no force is applied). The gather can be configured such that the inner portion of the side sheet 7 in the widthwise direction D2 is fixed to the top sheet 3, and the outer portion of the side sheet 7 in the widthwise direction D2 rises.

The absorbent article 101 can be configured to have no gather, as in the absorbent article 1 (first embodiment). Meanwhile, the absorbent article 1 (first embodiment) may be configured to include a gather.

Although the present invention has been described above based on the embodiments, the present invention is not limited to these embodiments. In addition, the above embodiments can be subjected to changes, modifications, substitution, addition, deletion, and combination in various ways within the scope recited in claims, and these are also within the technical scope of the present invention.

Hereinafter, specific aspects of the present invention will be described.

### (Clause 1)

In an aspect according to Clause 1, an absorbent article includes: a liquid-permeable top sheet; a liquid-impermeable back sheet; and an absorbent provided between the top sheet and the back sheet, the absorbent article having a longitudinal direction and a widthwise direction orthogonal to the longitudinal direction, in which the absorbent article includes a center compressed groove extending along the longitudinal direction in a center region in the widthwise direction, and a pair of oblique compressed grooves that are each connected to one end of the center compressed groove in the longitudinal direction and are extended such that a distance between the pair of oblique compressed grooves increases outward in the longitudinal direction, and a length of the oblique compressed grooves in the longitudinal direction is 30% or more a length of the center compressed groove in the longitudinal direction.

According to the aspect according to Clause 1, the compressed grooves (the center compressed groove and the pair of oblique compressed grooves) are formed in the absorbent article. With this configuration, the absorbent article is readily folded at the compressed grooves, and deformation into a desired shape can be facilitated upon wearing of the absorbent article. The pair of oblique compressed grooves are connected to one end of the center compressed groove, and thus deformation of the absorbent article can be guided continuously over the entirety in the longitudinal direction. Therefore, possibility of occurrence of undesired deformation, e.g., possibility of formation of unwanted wrinkles, twists, and the like can be reduced in the longitudinal direction, and leakage can be sufficiently prevented.

In the present aspect, the distance between the pair of oblique compressed grooves increases outward in the longitudinal direction. With this configuration, the center compressed groove and the pair of oblique compressed grooves form a compressed groove that is continuous in a Y shape. Therefore, natural deformation conforming to the shape of the wearer's body, especially the inner shapes of the legs is readily achieved. That is, the absorbent article is readily deformed such that a region corresponding to the groin of the absorbent article (the middle region in the longitudinal direction) becomes narrower and regions forward and/or rearward of that region becomes wider. Further, the length of the oblique compressed grooves in the longitudinal direction is 30% or more the length of the center compressed groove in the longitudinal direction. With this configuration, the above-described deformation conforming to the inner shapes of the legs can be more readily achieved, and the effect of preventing leakage can be enhanced.

### (Clause 2)

In an aspect according to Clause 2, a width of the oblique compressed grooves is smaller than a width of the center compressed groove.

According to the aspect according to Clause 2, the center compressed groove can be formed to have a larger width. With this configuration, the absorbent article is readily folded at the center compressed groove, and can be more reliably deformed such that the center of the absorbent article in the widthwise direction becomes convex with respect to the non-skin side (underwear side). Further, the oblique compressed grooves can be formed to have a smaller width, and thus it is possible to prevent a portion that touches the body in a relatively large area forward and/or rearward of the body, from becoming harder and to prevent discomfort felt, for example, upon wearing of the absorbent article.

### (Clause 3)

In an aspect according to Clause 3, each of the pair of oblique compressed grooves is curved to be convex with respect to a longitudinal centerline extending along the longitudinal direction.

According to the aspect according to Clause 3, the absorbent article can be naturally deformed to conform to the curved shapes around the legs.

### (Clause 4)

In an aspect according to Clause 4, the pair of oblique compressed grooves includes: a pair of forward oblique compressed grooves that are each connected to a forward end of the center compressed groove in the longitudinal direction and are extended such that a distance between the pair of forward oblique compressed grooves increases forward in the longitudinal direction; and a pair of rearward oblique compressed grooves that are each connected to a rearward end of the center compressed groove in the longitudinal direction and are extended such that a distance between the pair of rearward oblique compressed grooves increases rearward in the longitudinal direction.

According to the aspect according to Clause 4, continuous Y-shaped compressed grooves can be formed by the center compressed groove and the pair of oblique compressed grooves both of forward and rearward of the absorbent article. Therefore, the entire absorbent article can be readily deformed into a desired shape over the entire region from forward to rearward of the body.

### (Clause 5)

In an aspect according to Clause 5, both of the forward oblique compressed groove and the rearward oblique compressed groove on one side of the longitudinal centerline extending along the longitudinal direction are formed along the outline of a single identical imaginary circle, and a radius of the imaginary circle is from 70 mm through 100 mm.

According to the aspect according to Clause 5, it is possible to further improve the effect that the absorbent article can be deformed more naturally to conform to the curved shapes around the legs.

### (Clause 6)

In an aspect according to Clause 6, a sum of a length of the forward oblique compressed groove in the longitudinal direction, the length of the center compressed groove in the longitudinal direction, and a length of the rearward oblique compressed groove is from 40% through 75% a length of the absorbent in the longitudinal length.

According to the aspect according to Clause 6, the entire length of the compressed grooves is sufficiently ensured in the longitudinal direction of the absorbent article. With this configuration, it is possible to achieve desired deformation more reliably, prevent formation of wrinkles, twists, and the like at undesired sites, and achieve deformation conforming to the shapes of the legs.

### (Clause 7)

In an aspect according to Clause 7, the center compressed groove and/or the pair of oblique compressed grooves include a weakly compressed portion and a strongly compressed portion that is compressed deeper than in the weakly compressed portion, and the strongly compressed portion is discontinuously formed in the weakly compressed portion.

According to the aspect according to Clause 7, the weakly compressed portion and the strongly compressed portion are combined. With this configuration, deformation along the compressed grooves can be readily achieved, and flexibility of the compressed grooves can be ensured.

The present application claims priority to Japanese Patent Application No. 2021-176394, filed on October 28, 2021, the entire contents of which are incorporated herein.

### REFERENCE SIGNS LIST

1 absorbent article
2 back sheet
3 top sheet
4 absorbent
7 side sheet
11, 111 center compressed groove
11f, 111f forward end of center compressed groove
11r, 111r rearward end of center compressed groove
12, 112 forward oblique compressed groove
12f, 112f forward oblique compressed groove
12r, 112r rearward oblique compressed groove
C center region in widthwise direction
CL longitudinal centerline
D1 longitudinal direction (first direction)
D2 widthwise direction (second direction)
RE end region
RE1 forward end region
RE2 rearward end region
RM middle region
5 side region in widthwise direction
Q body fluid outlet-facing region

## Claims

1. An absorbent article, comprising:
a liquid-permeable top sheet;
a liquid-impermeable back sheet; and
an absorbent provided between the top sheet and the back sheet,
the absorbent article having a longitudinal direction and a widthwise direction orthogonal to the longitudinal direction, wherein
the absorbent article includes
a center compressed groove extending along the longitudinal direction in a center region in the widthwise direction, and
a pair of oblique compressed grooves that are each connected to one end of the center compressed groove in the longitudinal direction and are extended such that a distance between the pair of oblique compressed grooves increases outward in the longitudinal direction, and
a length of the oblique compressed grooves in the longitudinal direction is 30% or more a length of the center compressed groove in the longitudinal direction.

2. The absorbent article according to claim 1, wherein
a width of the oblique compressed grooves is smaller than a width of the center compressed groove.

3. The absorbent article according to claim 1 or 2, wherein
each of the pair of oblique compressed grooves is curved to be convex with respect to a longitudinal centerline extending along the longitudinal direction.

4. The absorbent article according to any one of claims 1 to 3, wherein
the pair of oblique compressed grooves include
a pair of forward oblique compressed grooves that are each connected to a forward end of the center compressed groove in the longitudinal direction and are extended such that a distance between the pair of forward oblique compressed grooves increases forward in the longitudinal direction, and
a pair of rearward oblique compressed grooves that are each connected to a rearward end of the center compressed groove in the longitudinal direction and are extended such that a distance between the pair of rearward oblique compressed grooves increases rearward in the longitudinal direction.

5. The absorbent article according to claim 4, wherein
both of the forward oblique compressed groove and the rearward oblique compressed groove on one side of the longitudinal centerline extending along the longitudinal direction are formed along the outline of a single identical imaginary circle, and
a radius of the imaginary circle is from 70 mm through 100 mm.

6. The absorbent article according to claim 4 or 5, wherein
a sum of a length of the forward oblique compressed groove in the longitudinal direction, the length of the center compressed groove in the longitudinal direction, and a length of the rearward oblique compressed groove is from 40% through 75% a length of the absorbent in the longitudinal length.

7. The absorbent article according to any one of claims 1 to 6, wherein
the center compressed groove and/or the pair of oblique compressed grooves include a weakly compressed portion and a strongly compressed portion that is compressed deeper than in the weakly compressed portion, and
the strongly compressed portion is discontinuously formed in the weakly compressed portion.
